# EUROPEAN PATENT APPLICATION

(11) **EP 2 842 885 A1**
(43) Date of publication of application: **04.03.2015**
(21) Application number: 13462003.8
(22) Date of filing: 02.09.2013
(51) Int. Cl.: B65D 51/20, C12M 1/24, C12M 1/00

(54) **A sampling vessel for automated sampling**

(71) Applicant: Bay Zoltán Közhasznú Nonprofit Kft., 1116 Budapest (HU)
(72) Inventor: Tolmacsov, Péter, H-6725 Szeged (HU)
(74) Representative: Szabo, Zsolt

(57) **Abstract**

The present invention relates to a sample-holder vessel (10), in particular for sampling operations executed by an automated sample feeder apparatus having a sampling needle. Said sample-holder vessel (10) has got a body (11) defining an inner space and comprises a top portion and a lower section, said body (11) is provided with a first sampling port (12) at the top portion and a second sampling port (14) in the lower section, said sampling ports (12, 14) are formed enabling a detachable connection with closing elements (13, 15) enabling subsequent sampling from predetermined regions of the inner space, the inner space of said sample-holder vessel (10) is closed in a leakproof manner by said closing elements (13, 15) when said closing elements (13, 15) are in the connected position. Moreover, a geometric axis of said first sampling port (12) runs substantially parallel to the sampling needle during sampling operations by the sampling needle from the sample-holder vessel (10) via the first sampling port (12). Furthermore, a collar (16) is arranged in the region of the first sampling port (12) on the external surface of the sample-holder vessel (10), said collar (16) forming a locating gap (17) in cooperation with the closing element (13) connected to the first sampling port (12) for securing the immobility of the sample-holder vessel (10) relative to the sample feeder apparatus during sampling operations via the first sampling port (12) by receiving a fixing element associated with the sample feeder apparatus and by a stationary fit of the sample-holder vessel (10) and the fixing element.

## Description

The present invention relates to a sample container, in particular, to a sample-holder vessel that can preferably be used in biological/biotechnological laboratory measurements. The sample-holder vessel according to the invention can highly preferably applied in those cases where the sampling from the internal space of said sample-holder vessel takes place in an automated way, for example through an automated sample feeder apparatus (from now on, "autosampler").

One of the most frequently used means of biological/biotechnological laboratory studies is the so-called microcosm or microcosm experiment. For this kind of measurement, systems of smaller or larger volumes are formulated in sample-holder vessels according to pre-defined aspects and then investigated for a period of time. If needed, the biological or chemical composition of the systems, the changes in their properties and/or, in general, the evolution in time of the systems are analyzed. The measurements are generally performed by making use of several microcosms at the same time, each microcosm is set up under fully identical initial conditions. Usually, the analysis is carried out via sampling said parallel microcosms. To this end, a sample is taken out from each part of the systems in various phases arranged in the sample-holder vessels at different pre-set instants as time progresses. Based on the data obtained through studying the samples, advance estimates can be made as to the evolution of various (system) processes on the macroscopic scale (e.g. in the environment). The microcosm experiments can well be used *inter alia* to model basic environmental problems under controlled circumstances, and/or to prevent or alleviate the environmental problems studied, various biological solutions can be suggested on the basis of their results.

In order that the estimates and the responses proposed on the basis of said estimates could be the possible most accurate, precision and high reproducibility of the microcosm experiments are essential. It is also important that said microcosm experiments model reality to the best possible way. Moreover, when parallel microcosms are used, it is essential that handling, e.g. sampling, of each micro-cosm - as far as possible - take place in the same manner in every single case.

A yet further basic question of the microcosm experiments is the size, and in turn, the practicability due to this. Here, this means the ease of handling within a series of experiments. As it was experienced, the microcosm experiments performed in small volumes, i.e. when e.g. headspace vials of at most 5 to 10 ml in volume capacity are used as the sample-holder vessels, are rather user-friendly: they can be handled with ease, their storage and insertion within incubators and agitating machines are not too problematic, even if a plurality of parallel samples is provided. Last but not least, the microcosms arranged in such headspace vials can be studied by direct headspace gas chromatographic analytical techniques. Said small volume, however, also has got disadvantages, e.g. when the microcosms are formulated, the possibility of mistakes is higher. Moreover, as it was also experienced, in case of systems of small volume, there is a high fluctuation among the parallel starting points that can significantly influence the evolution trajectories of said microcosms and, thus, the reliability of an estimation based on the analysis.

In case of larger experimental volumes utilized to enhance reliability, and especially when a plurality of independent, separate parallel microcosms are used/studied simultaneously, storage of the sample-holder vessels containing the microcosms poses a great problem. In such a case, to carry out consecutive samplings exactly the same way from each phase and, thus, generally from both the liquid and the gaseous phases, leads to a further problem. In light of this, automation of said sampling, i.e. its performing by an autosampler seems to be expedient.

U.S. Patent No. 3,793,154 discloses a flask for microcosm experiments which is equally suitable for establishing and maintaining aerobe and anaerobe environmental conditions. Said flask comprises a teflon-coated threaded tube equipped with a closing cap in its neck portion to accommodate the microcosm in the flask, a bottom, as well as a skirt portion connecting the tube in the neck portion with the bottom. Three laterally arranged threaded tubes protrude from said skirt portion. A septum-type silicon plug is inserted into each lateral tube to provide sealing of the tubes. At the ends of said lateral threaded tubes, there is provided each a threaded closing cap; each cap has got a hole in it to allow the passage of a hypodermic needle therethrough. One of the lateral threaded tubes serves to feed a gaseous substance into the interior of the flask. A further threaded tube functions to sample the liquid phase of the microcosm disposed in the flask, while the third lateral threaded tube is adapted to be suitable for connecting to a sample analyzer to perform e.g. a headspace chromatography analysis (i.e. to sample the gaseous phase of said microcosm).

The drawback of said flask derives, on the one hand, from its relatively complicated design (providing three lateral threaded tubes in the skirt portion), and, on the other hand, from the practical realization of the tube for sampling the gaseous phase: this latter element is basically perpendicular to the vertical axis of said flask, and thus is ill-fitted to cooperate with the autosamplers available nowadays. Moreover, as said tube projects out from the skirt portion to a given length, optionally, one or more gaseous components of the gaseous phase of the microcosm can condense in it due to the heat exchange through the wall of said tube. As a consequence of effecting a change in the composition of the gaseous phase, this can affect the results obtained by the analysis of said gaseous phase and, in an extreme case, can result in arriving at erroneous conclusions.

Reliability of the results obtained through microcosm experiments can be enhanced if the measurement, and then the analysis is performed in each microcosm separately at consecutive given instants of the test period, and thereby a temporal evolution of every single microcosm used becomes available. This is, however, possible only in that case if the sampling does not disturb the microcosm in the sample-holder vessel (e.g. via a non-desired contamination). To this end, the sampling tubes of the sample-holder should be provided with a closing means that, on the one hand, allows multiple samplings, and on the other hand ensures that the conditions prevailing in the microcosm having just been sampled remain basically unchanged during the sampling and after it. U.S. Patent Nos. 3,603,471 and 4,187,149 disclose a closing means with a single and two pieces of, respectively, elastic septum member that can be punctured many times by a sharp means, e.g. by a needle mounted onto the end of a syringe to inhibit any communication between the internal space of the sample-holder vessel and the external surroundings. In these cases, said septum members are always located in properly adapted septum holders that borrow adequate mechanical support to the septum members used. The thus obtained closing means, nevertheless, are rather complicated as to design and, hence, their production costs are relatively high.

For a gas chromatography analysis of the gaseous phase of the microcosm, it is a rather important requirement to always withdraw a sample of precisely the same volume from the gas plenum of the sample-holder vessel at consecutive samplings. Or putting this another way, reproducibility/repeatability of the sampling of the gaseous phase is of basic importance. As far as microcosm experiments are concerned, in harmony with this, an automated sampling seems to be highly expedient

The operation principle of autosamplers used for automated sampling is as follows: a given amount of sample is withdrawn from the sample within the sample-holder vessel arranged in a certain position on a sample-holder tray representing, in general, a standard accessory of the autosampler apparatuses by means of an (generally cusped/sharp) injecting needle that is displaced along a predetermined path, and then to analyze further the thus obtained sample, it is fed into the inlet duct of a dedicated apparatus for performing the study (e.g. a gas or liquid chromatography apparatus). At sampling and at sample discharge, the tip of the injecting needle is located at two well-defined locations of space, and moves between these end points along a pre-defined path. The possible spatial coordinates of said end points are programmed into the control device of the autosampler. If the injecting needle tries to perform the sampling and/or the discharge of the sample at locations that differ from the end points, the needle could be damaged. This poses an extraordinary huge problem in case of the so-called armed autosamplers, wherein the injecting needle is located at the end of a robotic arm adapted to be displaced, at least in one spatial direction, between certain limiting positions. This problem enhances further if the autosampler is to be used for sampling such sample-holder vessels that are not the standard accessories provided by the producer of the autosampler, as can be the case for sample-holder vessels of larger volume (at least 50 ml in volume capacity) utilized e.g. in the microcosm experiments.

In light of the above, an object of the present invention is to provide a sample-holder vessel, especially to perform microcosm experiments, that allows for multiple samplings of the gas plenum forming above a (micro)biological system with solid and/or liquid phases in said vessel in a repeatable way. In particular, another object of the present invention is to provide a sample-holder vessel that can be sampled by an automated sample feeder apparatus, that is by an autosampler, to ensure multiple samplings of said gas plenum along with withdrawing the same sample volumes, i.e. repeatability (reproducibility) of said sampling. A yet further object of the present invention is to provide a sample-holder vessel that, as a consequence of its design, is suitable for monitoring and/or determining a precise and complete temporal evolution of the microcosm studied via performing sampling many times, consecutively on the microcosm disposed therein. Here, and from now on, the study of a "complete temporal evolution" refers to the simultaneous, parallel study of a (micro)biological system with solid and/or liquid phases, as well as of the gas plenum that forms above said system.

The above objects are achieved by providing a sample-holder vessel, in harmony with the features of Claim 1, that is suitable for being sampled in its gas plenum and liquid plenum simultaneously due to its construction, according to which the liquid-sampling location is formed on the side wall of the sample-holder vessel and the mouth opening of said sample-holder vessel is adapted to be capable of sampling the gas plenum. In particular, said mouth opening of the sample-holder vessel is equipped with such a gastight/leakproof closing means that, on the one hand, ensures multiple sampling of the gas plenum in a reproducible manner and, on the other hand, by cooperating with the sample-holder vessel, facilitates the positioning of the sample-holder vessel required for the sampling and its immobility when being sampled. As a result of the cooperation between said closing means and the sample-holder vessel, the sample-holder vessel according to the present invention becomes also suitable to be used with a sample feeder apparatus for automated sampling. To this end, the application of an appropriate tray adapter is required; a certain portion of said adapter tray enters a locating gap that forms as a consequence of the cooperation between the closing means and the sample-holder vessel, and thereby ensures the immobility of said sample-holder vessel during its sampling. That is, by arranging a tray adapter on/in the sample-holder tray provided as standard accessory for an autosampler, desired positioning of the sample-holder vessel can be ensured.

Possible further embodiments of the sample-holder vessel according to the invention are set forth by Claims 2 to 9.

Furthermore, as the liquid-sampling location is below the liquid level of the (micro)biological system accommodated in the vessel when the microcosm experiments representing the main application field of the sample-holder vessel according to the invention are actually taken place, sampling of the liquid plenum of said vessel can be performed without, on the one hand, the component in the liquid plenum being stirred up and, on the other hand, making use of an expelling gas that affects the composition of the gas plenum.

Moreover, in the microcosm experiments, both the gas plenum and the liquid plenum can be sampled in a closed state of the sample-holder vessel, i.e. without breaking up the vessel and thus changing/affecting the composition at least in the gas plenum. Consequently, to perform a series of experiments, there is a need for significantly fewer parallel microcosms compared to what is used nowadays, and/or some of the parallel microcosms can be used for checking and/or statistical studies/analysis.

By properly choosing the volume of the sample-holder vessel to perform the microcosm experiments, the (aliquot) liquid and/or gas volumes withdrawn for sampling from the (micro)biological system accommodated in the sample-holder vessel will not disturb the system under study even if an extended experiment comprising, in general, more than ten, preferably at least fifteen, more preferably at least twenty consecutive samplings takes place. Or putting this another way, as a consequence of sampling the (micro)biological system accommodated in a sampler-holder vessel according to the present invention by an autosampler, even if unusually high number of samplings take place, it does not influence the measuring results significantly.

Based on the more accurate results obtained in microcosm experiments performed with making use of the sample-holder vessel according to the invention, the processes in macroscopic systems, as well as the answers to be given therefore can be estimated much more reliably. This is partially due to the fact that when a sample-holder vessel according to the invention is utilized, instead of using parallel microcosms, the one and same microcosm can be subjected to the study.

In what follows, the invention is discussed in more detail with reference to the attached drawings, wherein
- Figure 1 illustrates schematically a preferred embodiment of the sample-holder vessel according to the invention;
- Figure 2 is a longitudinal sectional view of the mouth opening of the sample-holder vessel shown in Figure 1 and the closing means provided as a closing cap that closes said mouth opening in an airtight manner;
- Figure 3 is an exploded view of a possible further embodiment of the sample-holder vessel shown in Figure 1;
- Figure 4 illustrates schematically a tray adapter that allows for using the sample-holder vessel according to the invention in combination with an autosampler; and
- Figures 5A and 5B show the temporal evolution (anaerobe degradation) of a biological system, in particular a system of soil/ground water polluted with halogenated aliphatic hydrocarbon compounds, in light of the results obtained by sampling the internal space of the sample-holder vessel.

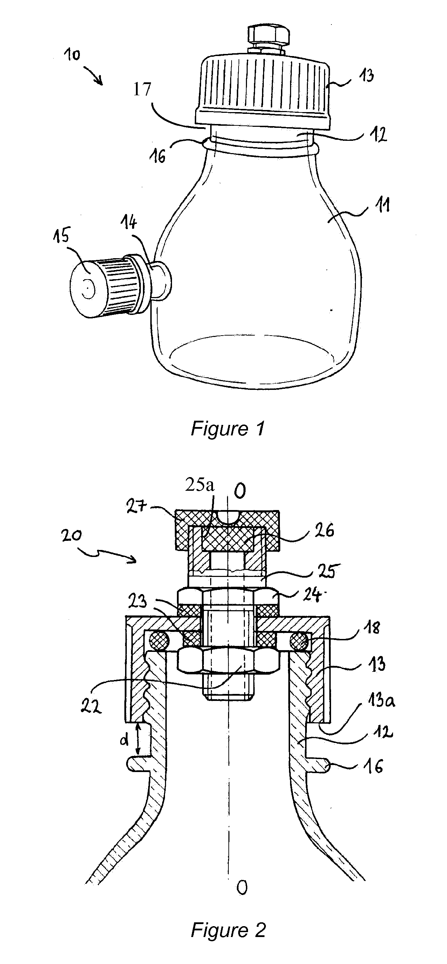

Figure 1 shows a sample-holder vessel 10 according to the present invention that comprises a body 11 defining an internal space and a sampling port 14 that opens into said internal space (preferably, in a lower portion of the body 11). Said body 11 further comprises a top portion with a mouth opening 12 therein. In the assembled state of the sample-holder vessel 10, the mouth opening 12 and the sampling port 14 are covered by closing caps 13 and 15, respectively. In this embodiment, to connect the closing caps 13, 15 forming closing means via respective threads formed around the outer sides of the mouth opening 12 and the sampling port 14, said closing caps 13, 15 are provided with respective matching threads around their inner sides. Here, the term "respective threads" refer to the fact that said threads are constructed with pitches that enable proper closure (i.e. leakproof closing). For a skilled person in the art it is apparent that, in practice, this requirement corresponds to fine pitches. Consequently, the closing caps 13, 15 are detachably connected to the sample-holder vessel 10, and when they are connected (i.e. "screwed on"), they provide airtight closing of the internal space of the sample-holder vessel 10. Furthermore, the sample-holder vessel 10 is provided with a collar 16 on its external surface at a given distance from the top of said mouth opening 12. Preferably, said collar 16 is made of the material of the body 11 and is formed when the body 11 is produced, through its moulding, however, this is not necessary; the collar 16 can be equally formed on the external surface of the body 11 at the location of usage as well by attaching (e.g. bonding) a suitable material to said body 11. Said collar 16 can be realized as a single continuous element extending around the external surface of the sample-holder vessel 10, or alternatively it can be formed as several separate projections apart from each others.

In the embodiment shown here, (apart from the port 14) the body 11 has preferably a rotation-symmetric shape, but this is not a requirement. Furthermore, in this embodiment, the mouth opening 12 is formed so as to be located in the highest portion of the body 11 (at its top portion) and to occupy essentially a central position; however, the latter is not a requisite either. The mouth opening 12 functions as a gas sampling location for the internal space of said sample-holder vessel 10 (that is, it serves as a sampling port that allows for sampling the gaseous components accumulating in the sample-holder vessel 10 during e.g. a microcosm experiment), as it will be discussed in more detail later on. In the embodiment shown here, the port 14 is preferably arranged in the flared lower portion of the body 11; as far as its accurate position is concerned, it can be stated that said port 14 is arranged on the body 11 in such a way that liquid-type sampling of the internal space of the sample-holder vessel 10 could be performed through the port 14 without influencing gaseous components within the sample-holder vessel 10. In the embodiment shown here, from production engineering considerations, basically the geometrical axis of said port 14 is perpendicular to the geometrical axis of said mouth opening 12 (or it forms an angle of at most several degrees with a line that is perpendicular to the geometrical axis of said mouth opening 12).

Figure 2 illustrates the mouth opening 12 of a preferred embodiment of the sample-holder vessel according to the invention in the longitudinal sectional view, with the closing cap 13 screwed onto it. The closing cap 13 made preferably of polypropylene (PP) is adapted to allow for sampling the internal space of the sample-holder vessel and by inserting an annular sealing member 18 of given thickness it closes said space in an airtight manner. To perform sampling, the closing cap 13 is equipped with a sampling assembly 20. Said sampling assembly 20 is provided by a valve unit assembled basically from separate elements; said sampling assembly 20 can detachably and thus replaceably be installed into a through hole of a given size that is formed in the top face of said closing cap 13. The elements of the sampling assembly 20 are themselves replaceable elements, hence the valve unit concerned can easily be repaired, and in case of need (even on an element by element basis) replaced.

Besides its sealing function, said sealing member 18 has also got a spacer function in the sample-holder vessel 10 according to the invention. Consequently, its thickness is chosen in such a way that a bottom edge 13a of the closing cap 13 is situated from the collar 16 a distance d apart when said closing cap 13 is screwed onto the mouth opening 12. In this way, a locating gap 17 with height d is formed between the closing cap 13 and said collar 16 as a result of their cooperating behaviour. This locating gap 17 allows for using said sample-holder vessel 10 with an autosampler, as it will be discussed in more detail later on. The sealing member 18 is made of a resilient material that preferably also possesses chemical resistance; preferably, it is made of teflon (PTFE) that further enhances the airtightness and leakproofness of said closure with the closing cap 13. As it is apparent to a person skilled in the art, the sealing member 18 can be made of other suitable materials as well. Furthermore, in a possible further preferred embodiment, the sealing member 18 can be constructed as an integral part of the closing cap 13; in such a case, for example, the closing cap 13 itself is made of PTFE.

The major element of said sampling assembly 20 is a preferably cylindrical, tube shaped septum holder 25 that has a recess 25a of given depth at one of its ends along the direction of its geometrical axis O, wherein a flange 24 extends around the external, preferably cylindrical skirt of said septum holder 25. Said flange 24 is located at about the half of the septum holder 25, it is preferably formed in the material of the septum holder 25 by mechanical machining (e.g. by lathe machining) of its external surface at the time of producing said septum holder 25. At the other end of the septum holder 25, a thread (not shown in the drawings) is formed to receive a retaining nut 22. The inner diameter of the septum holder 25 is at most about several mm in size, it basically corresponds to the outer diameter of the sampling needles extensively used in the relevant art; when sampling is performed, the sampling needle can penetrate the inner hole of the septum holder 25 with clearance, said inner hole of the septum holder 25 guides the sampling needle unobstructedly into the internal space of the sample-holder vessel 10. Said septum holder 25 is made of an inert material that also possesses chemical resistance; preferably it is made of stainless steel.

A septum 26 is arranged within the recess 25a of the septum holder 25. The septum 26 can be provided e.g. by a traditional chromatographic septum. The elastic material of said septum 26, on the one hand, completely fills the recess 25a, it is arranged with positive locking in the recess 25a, and on the other hand, due to its elasticity, it enables that the sampling needle could enter the internal space of the sample-holder vessel 10 through it several times when sampling is performed along with maintaining the gas-proof closure. Said septum 26 is kept fitted into the septum holder 25 by a closure lid 27 that is mounted onto the septum holder 25 in a detachable manner. To pass through said sampling needle, the closure lid 27 is provided with a central aperture or a weakened portion. As the closure lid 27, for example a closure lid listed in the catalogue of Agilent Technologies Inc. (under item no. 18740-60835) can preferably be used. Preferably, the configuration of the end of the septum holder 25 with the recess 25a (with the closure lid 27 mounted thereon) is similar to that of gas chromatography inlets extensively used in the field of analytical chemistry. Consequently, the sample-holder vessel 10 according to the invention can optionally be connected to a gas chromatography apparatus in a direct manner as well.

Important additional parts of said sampling assembly 20 are the sealing members 23 that are arranged between the flange 24 and the retaining nut 22 on opposite sides of the top face of the closing cap 23 when said sampling assembly 20 is installed into the closing cap 13. Said sealing members 23 are made of an elastic material, preferably teflon, and have thicknesses of at most 1 mm, preferably at most 0.5 mm. Said sealing members 23 enable gas-proof installability of the sampling assembly 20 into the closing cap 13.

Fixing of the sampling assembly 20 into the closing cap 13 is ensured by the retaining nut 22 that can be screwed on the threaded end of the septum holder 25. In the present embodiment, the retaining nut 22 is made of stainless steel, and thus it is resistant against the reactive medium/media optionally present in the internal space of the sample-holder vessel 10. It is apparent to a person skilled in the art that the retaining nut 22 can also be made of any further materials that satisfy the prescribed mechanical and/or chemical resistance requirements.

Furthermore, it is noted hereby that in a possible further embodiment, the sampling assembly 20 and the closing cap 13 can also be provided as a single integrated unit, if an appropriate choice of material is made.

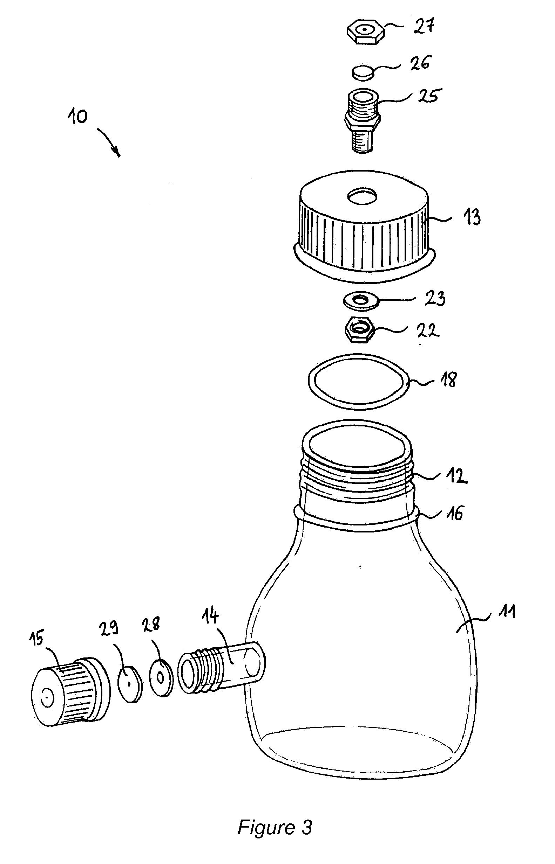

Figure 3 shows schematically the port 14 representing the liquid sampling position of the sample-holder vessel 10, as well as the constructional elements that provide the liquid-proof closing of said port 14. According to this, the opening of the port 14 is closed by a well-known gas chromatography septum 29 arranged on an annular septum support 28 which are kept in position by the closing cap 15 screwed onto the thread formed on the external surface of said port 14. To enable penetration of a sampling needle, said closing cap 15 has got a central opening or a weakened portion in its top face. The septum support 28 acts as a supporting member to support the septum 29 that basically exhibits no mechanical strength. Consequently, said septum support 28 has got adequate mechanical strength and is preferably made of an inert material, preferably teflon. The closing cap 15 is preferably provided by a closing cap made of polypropylene. It is apparent to a person skilled in the art that the closing cap 15, the septum 29, and the septum support 29 can also be provided as a single integrated unit, if an appropriate choice of material is made.

It is noted that the port 14 can also be adapted to receive a sampling assembly similar to the sampling assembly 20. In such a case, the construction of the closing cap 15 is basically identical to that of the closing cap 13. However, the configuration shown in Figure 3, enabling (liquid-type) sampling through the port 14 constitutes a much cheaper and simpler solution along with ensuring proper tightness of the port 14.

It is also noted that in the embodiment of the sample-holder vessel 10 according to the invention illustrated in Figure 3, one of the sealing members 23 is formed as part of the septum holder 25, in particular, it constitutes an integral unit with said flange 24 of the septum holder 25 (on that side of the flange 24 which faces the closing cap 13).

As it has been previously discussed, the sample-holder vessel 10 is especially preferred for studying the complete temporal evolution of a microcosm disposed in its internal space. To this end, sample is taken from the internal space of said vessel 10 from time to time. Sampling is performed from the gaseous phase filling the gas plenum above the (micro)biological system accommodated within the sample-holder vessel 10 through the sampling assembly 20, and from the substance of the (micro)biological system itself through the port 14 having a liquid-proof closure, preferably simultaneously, and to ensure reproducibility to a possible highest extent, by completing sampling from the gaseous phase with an automated sample feeder apparatus, via its sampling needle. To this end, the sample-holder vessel 10 is arranged in a tray adapter 30 that matches said sample-holder vessel 10 with the autosampler, in particular with an armed-type autosampler to be applied. By means of a suitable tray adapter 30, the standard test tray of an autosampler with factory tray can easily and rapidly be transformed into a tray that allows for sampling the gaseous phase in an automated and, hence, well reproducible manner by fixing said sample-holder vessel 10 according to the invention.

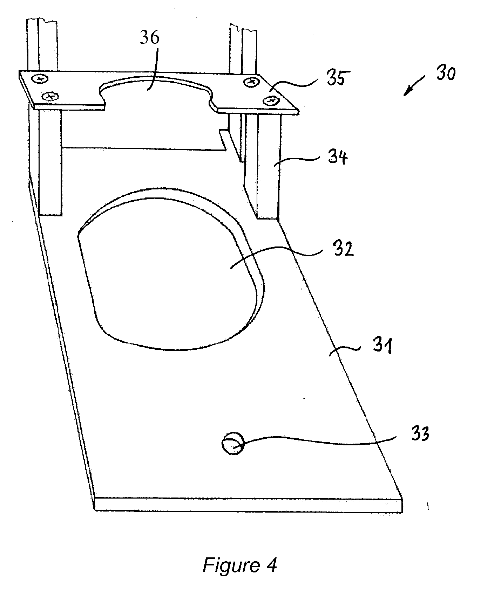

A preferred embodiment of the tray adapter 30 is shown in Figure 4. It is apparent to a skilled person in the art that the embodiment of the tray adapter 30 shown in Figure 4 represents only a possible exemplary embodiment of said tray adapter, the actual structural design of which can be varied/scaled as a function of the real dimensions of the autosampler to be used. This exemplary embodiment, however, reflects a basic concept of the present invention, namely, to perform sampling in an automated manner, the sample-holder vessel can reliably be fixed with the suitable tray adapter 30 due to a cooperation of the closing cap equipped with the sampling assembly and said sample-holder vessel. To sample the gaseous phase by the autosampler in an automated manner, it is essential that the position of the sample-holder vessel relative to the autosampler is fixed at the moment of sampling, i.e. the sample-holder vessel could not move relative to the autosampler to avoid damaging of the sampling needle of the autosampler.

The structure of tray adapter 30 is very simple: it contains a base plate 31 that can be arranged in/on the test tray of the autosampler and a clamping plate 35 supported by columns 34 set on the base plate 31. Columns 34 and clamping plate 35 may be fixed to the base plate 31 and the columns 34, respectively, in an arbitrary way, but fixing by screws in bores prepared in the base plate 31, columns 34 and clamping plate 35 is considered to be particularly advantageous to allow transformability necessary to use it with different types of autosamplers. The clamping plate 35 is provided with a cutout 36. The shape of the cutout 36 corresponds to the shape of a planar cross section of the neck of the sample container. More precisely, the cutout 36 has a shape and size suitable for receiving substantially unimpededly the area (see Figure 2) between bottom edge 13a of the closing cap 13 and the collar 16 of the body 11, under normal operating conditions (in other words, the sample-holder vessel 10 does neither move freely nor get stuck, that is there is a stationary fit between them); in this case, the term "normal operating conditions" means a temperature range between approx. -10°C and approx. +60°C. As it is obvious to the person skilled in the art, this range can be extended considerably in both directions by proper material selection, while the proper stationary fit is maintained.

In addition, the base plate 31 is also provided by a positioning hole 33 as operably required by the majority of the autosamplers, and a container receiving aperture 32 having a shape matching the base area of the a sample holder according to the present invention. The positioning hole 33 serves as to adjust the relative position of the base plate 31 and the autosampler, while the opening 32 is for supporting the sample holder. The height of the columns 34 substantially corresponds to a distance measured between the bottom and collar of the sample container intended to be used actually. At the same time, under normal operating conditions, the thickness of the clamping plate 35 is substantially equal to the height d of a locating gap 17 formed between the bottom edge 13a of the cap 13 and the collar 16, due to a cooperation of the cap 13 and the collar 16.

For an automated sampling of the gas space in the sample container 10 the sample holding container 10 is forwarded into the container receiving slot 32 of the tray adapter 30 by a sliding motion directed from front to back. Simultaneously, the clamping plate 35 slides into the locating gap 17 between the collar 16 of the sample holding container 10 and the edge 13a of the cap 13. In this situation the sample holding container 10 is maintained in fixed position by a stationary fit occurring between the sample holding container 10 and the clamping plate 35 (due to the friction between the two elements). Maintaining a fixed position is enhanced by a support effect exerted by the receiving slot 32 of the base plate 31 in the lower portion of the sample-holder vessel 10. After achieving the fixed position in question, the sample-holder vessel 10 is ready for automated sampling to be performed by the autosampler in a known manner.

Some advantages of applying the sample-holder vessel 10 according to the present invention are further illustrated by a particular example below.

### Example: study of the anaerobe degradation of halogenated aliphatic hydrocarbon compounds.

The degradation of soil and groundwater during bioremediation is carried out microbiologically. In order to use a remediation technology designed for bioremediation, preliminary laboratory experiments and/or studies are required. In most cases the materials polluting the soil and/or groundwater are volatile or semi volatile hydrocarbon compositions, which are compounds of different chemical properties and biodegradability. The biodegradation of a hydrocarbon composition is carried out on different metabolic routes and results in intermediates and end products of different phases.

The quantitative and qualitative analysis of intermediates (alcohols, partially reduced hydrocarbon derivatives, etc.) originating from the partial degradation of the starting hydrocarbon composition and composing compounds and of the end products (carbon dioxide, simple organic acids, methane, ethylene, etc.) has to be studied in parallel, simultaneously in the same (single) experimental system. Or putting this another way, simultaneous analysis of solid, liquid and gaseous hydrogen derivatives is required. Accordingly, the most accurate conclusions about the processes taking place in the studied sample can be deduced from such an experimental system, which allows for sampling from both the liquid and the gaseous phases without disturbing the system.

In order to carry out such a study, we have tested the effectiveness of chemical and microbiological reduction in soil/groundwater systems (microcosm) polluted by 1,2-dichlore-ethane using the sample-holder vessel according to the invention. We used FeO for chemical reduction and the biodegradation was induced by electrodonor (acetate) and Fe³⁺ reducing bacteria. As a control system we used killed (abiotic) and untreated (biotic) samples also placed in the sample-holder vessel according to the invention. We have assembled the systems under oxygen-free conditions and incubated them for 22 days at the temperature of 20°C. We have studied the rate of chemical reduction and biodegradation by gas chromatographic analysis, formation of carbon dioxide, methane, ethane and ethylene during biodegradation was observed and monitored by simultaneous sampling of the gaseous phase and the liquid phase present in the sample-holder vessel.

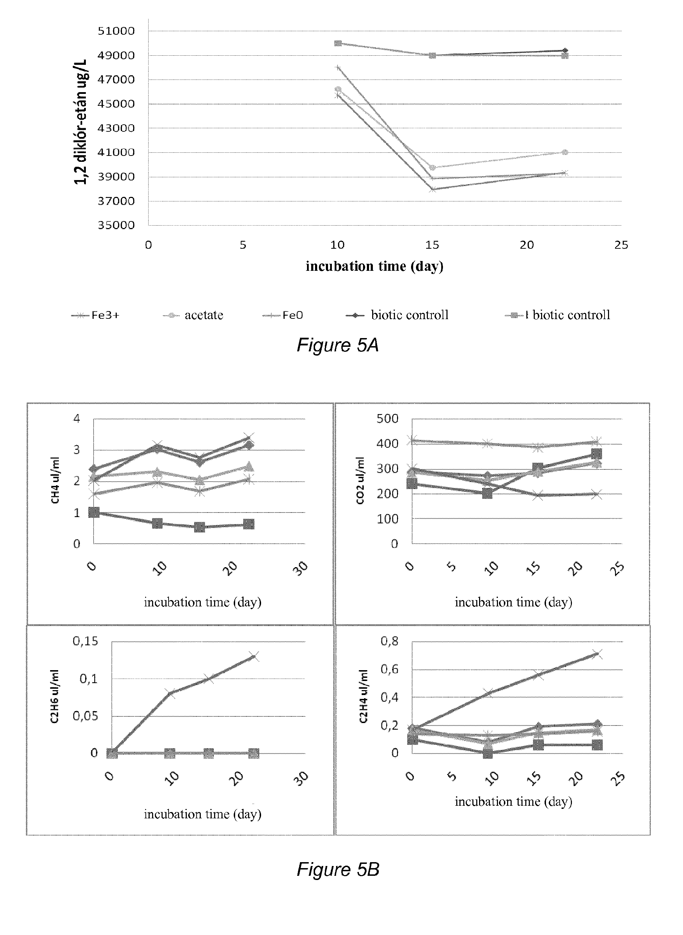

The results of the continuous studies are summarized in figures 5A and 5B. Figure 5A illustrates the measured hydrocarbon-composition in the liquid phase within the different experimental systems arranged in the sample-holder vessels according to the invention, while figure 5B illustrates the measured hydrocarbon-composition in the gas plenum thereof as the function of the incubation time.

Depending on which type (chemical or microbiological) of reduction or oxidation took place, different hydrocarbon-composition was experienced. The decrease in the substrate compound (1,2-dichlore-ethane in the present case) alone is not sufficient to conclude on the type of process of transformation, but the process became unambiguously identifiable based on the change of composition of the gas plenum. Based on this knowledge, it became possible to determine what kind of treatment will/can be suitable for remediating the given area.

As it is obvious to a person skilled in the art, the exemplary preferred embodiment of the sample-holder vessel according to the invention previously described in detail may be modified in many ways. For example if performing a study requires flushing the gas plenum, the sample-holder vessel can be provided with at least two additional tubes, which are preferably formed on the side wall of the sample-holder vessel and open into the gas plenum, and are provided with airtight closing means enabling the flushing. In a preferred embodiment these closing means can be formed according to the aforementioned way.

The body of the sample-holder vessel according to the invention has rotational symmetry (around the O geometric axis), and its volume is preferably between about 50 ml and about 1000 ml, more preferably 250-500 ml. If a larger sample-holder vessel is required, preferably having a volume of several litres, the body of the sample-holder vessel according to the invention is preferably made in an asymmetrical form. In this case, the gas sampling position arranged on the top portion of the vessel is displaced from a central position to a terminal position to allow its use together with the autosampler. The sampling tube ensuring the liquid sampling of the sample-holder vessel in this case is also on the bottom region of the body of the sample-holder vessel. Due to this, the gaseous and the liquid sampling of the sample-holder vessel according to the invention can be performed independently and without disturbing the corresponding phases in any way. Using these principles the vessel-height restrictions typical in the case of autosamplers can be simply evaded while retaining all advantages of automatic sampling.

Furthermore, the sample-holder vessel according to the invention - if needed and desired - can be equipped with further additional means, for example by incorporating it into the glass or polymer building up the wall of the vessel or by the external (for example threaded) tubes closable in a gastight manner and opening into the gas or liquid plenum. Such a means can be for example a pH-meter, which specifically allows for monitoring of the pH value of the microcosm disposed in the sample-holder vessel.

It is also apparent to a person skilled in the art, that the sample-holder vessel according to the invention can also be used in studies other than what was discussed above in detail, which require studying including sampling (i.e. for chromatography) a gas plenum created above a liquid or solid phase matter. This way, in particular, the evaporation of solid matter, in particular, sublimation processes thereof, and their temporal evolution can be studied, too.

As it is obvious, the sample-holder vessel according to the invention can be manufactured simply and at a low cost and together with the described tray adapter it can be used reliably with known autosamplers nowadays. We also note, that the sampling of the sample-holder vessel according to the invention while can be carried out automatically by an autosampler - if needed and desired - it can also be carried out manually..

## Claims

1. A sample-holder vessel (10), in particular for sampling operations executed by an automated sample feeder apparatus having a sampling needle, said sample-holder vessel (10) having a body (11) defining an inner space and comprising a top portion and a lower section, said body (11) is provided with a first sampling port (12) at the top portion and a second sampling port (14) in the lower section, said sampling ports (12, 14) are formed enabling a detachable connection with closing elements (13, 15) enabling subsequent sampling from predetermined regions of the inner space, the inner space of said sample-holder vessel (10) is closed in a leakproof manner by said closing elements (13, 15) when said closing elements (13, 15) are in the connected position, wherein a geometric axis of said first sampling port (12) runs substantially parallel to the sampling needle during sampling operations by the sampling needle from the sample-holder vessel (10) via the first sampling port (12), furthermore, a collar (16) is arranged in the region of the first sampling port (12) on the external surface of the sample-holder vessel (10), said collar (16) forming a locating gap (17) in co-operation with the closing element (13) connected to the first sampling port (12) for securing the immobility of the sample-holder vessel (10) relative to the sample feeder apparatus during sampling operations via the first sampling port (12) by receiving a fixing element associated with the sample feeder apparatus and by a stationary fit of the sample-holder vessel (10) and the fixing element.

2. The sample-holder vessel (10) according to claim 1 wherein said closing element (13) comprising a closing cap (13) provided with a sampling assembly (20).

3. The sample-holder vessel (10) according to claim 2 wherein the sampling assembly (20) is formed as a cylindrical tube-shaped element; at one of the ends of the tube-shaped element a longitudinal recess (25a) of given depth having a diameter exceeding the inner diameter of the tube-shaped element is formed; a septum (26) completely filling the cross-sectional area of the recess (25a) is arranged within the recess (25a); furthermore, the tube-shaped element has a threaded outer surface at its other end.

4. The sample-holder vessel (10) according to claim 3 wherein the sampling assembly (20) is fixed by a retaining nut (22) screwed on the threaded region of the tube-shaped element in an open-end hole formed in the upper face of the closing cap (13).

5. The sample-holder vessel (10) according to claim 2 wherein the sampling assembly (20) and the closing cap (13) are provided as a single integrated unit.

6. The sample-holder vessel (10) according to any one of claims 1 to 5 comprising a tube-shaped element with an inner diameter of at most about several mm in size.

7. The sample-holder vessel (10) according to claim 6 wherein the inner diameter of tube-shaped element is substantially identical with the outer diameter of the sampling needle of the sample feeder apparatus.

8. The sample-holder vessel (10) according to any one of claims 1 to 7 having a rotation-symmetric shape and a capacity of at least 50 ml and not more than 1000 ml, more preferably at least 250 ml and not more than 500 ml.

9. The sample-holder vessel (10) according to any one of claims 1 to 8 comprising further a fixing element constituting part of a tray adapter (30) having a form fitting into a sample holder tray constituting factory accessory of the sample feeder apparatus.
